# EUROPEAN PATENT SPECIFICATION

(11) **EP 0 861 594 B1**
(45) Date of publication and mention of the grant of the patent: **11.04.2001**
(21) Application number: 98200253.7
(22) Date of filing: 28.01.1998
(51) Int. Cl.: A22B 5/04

(54) **Method and device for taking a blood sample of a slaughtered animal.**
Verfahren und Vorrichtung zur Abnahme einer Blutprobe von einem Schlachttierkörper
Procédé et dispositif de prélèvement d'un échantillon de sang d'un animal abattu

(30) Priority: 31.01.1997 NL 1005157
(43) Date of publication of application: 02.09.1998
(73) Proprietor: NEDERLANDSE ORGANISATIE VOOR TOEGEPAST-NATUURWETENSCHAPPELIJK ONDERZOEK TNO, 2628 VK Delft (NL)
(72) Inventor: Logtenberg, Harry, 3721 JV Bilthoven (NL); Wijngaards, Gerrit, 3972 GS Driebergen (NL); van der Hoorn, Rudolf Johannes Gerardus Antonius, 5672 BK Nuenen (NL)
(74) Representative: Mertens, Hans Victor

(56) References cited:
- WO-A-90/14013

## Description

The invention relates to a method for taking a blood sample of an individual slaughter animal, such as a pig, cow, sheep or the like, according to the preamble of claim 1.

It is usual to sample the blood of animals destined for slaughter at the farm where they are reared. The blood sample obtained in this way, which can be traced back unequivocally to a specific animal by means of an earmark identification on the animals, is analyzed in a laboratory for abnormalities which could lead to the possibility of taking measures to withdraw the animal in question from slaughter and consumption or even to the rejection of all the livestock present at the farm for slaughter and consumption.

Although blood sampling is a necessary action for protecting public health, the operation of taking the blood samples at the farm is laborious, time-consuming and expensive, since to do this experts have to visit many farms every day and have to take and store blood samples by hand, blood samples have to be transported specially and extensive administration is required.

The object of the invention is to simplify considerably the blood sampling of slaughter animals and the administration thereof and to shorten drastically the time required for this purpose. Another object of the invention is to provide a method and device which enable the blood sampling to be mechanized.

The above objects, and other objects, are achieved using the method according to the invention as defined in claim 1. Sticking the slaughter animal is the action in the slaughterhouse in which a blood vessel of a slaughter animal, which has been stunned beforehand, is cut using a knife, so that the slaughter animal bleeds to death, as disclosed in WO-A-90/14013. At the moment of sticking, a sample of the stream of blood released can be taken easily and without loss of time.

Each sample reservoir, which only has to be able to contain a very small quantity of blood, forms a unit with the knife, which as a result can be handled in the conventional way by a sticker in a slaughterhouse for sticking an animal for slaughtering.

If the blood sample is collected in two different sample reservoirs which are coupled to the knife, one of these sample reservoirs can be analyzed while the other sample reservoir, which contains blood from the same slaughter animal as the first sample reservoir, can be stored for a possible second opinion.

In a simple and inexpensive embodiment of the method according to the invention, after detaching each blood-filled sample reservoir from the knife, the knife is cleaned and disinfected, and then (a) new sample reservoir(s) is/are coupled to the knife. By connecting the sample reservoir to the knife in a detachable manner, the knife, after taking a blood sample, can be quickly cleaned and made ready for sticking and taking a new blood sample from a subsequent slaughter animal. This also avoids cross-contamination of slaughter animals.

Preferably, one sample reservoir, which has been filled with blood during the sticking of the slaughter animal, is sealed after it has been detached from the knife and is conveyed to a room which is situated in the slaughterhouse and where one or more predetermined analyses are carried out on the blood sample. In this way, the blood sample is transferred to a "clean" room in order to be analyzed, the risk of the blood sample becoming contaminated or infected being minimal. In addition, it is thus possible for the blood sample to be analyzed within a very short time, so that the analysis results are known even during the further slaughtering process of the slaughter animal from which the blood sample originates, and in particular before the slaughter animal is cooled in a cooling chamber. If the blood sample analysis shows that this is necessary, the slaughter animal can be removed from the slaughter line.

A particularly inexpensive, simple and rapid way of transporting the sample reservoir takes place in a hose or tube with the aid of a fluid at superatmospheric pressure, such as compressed air, or at subatmospheric pressure.

The cleaning and disinfection of the knife after each sample reservoir filled with blood has been detached expediently comprises the following steps: cleaning with cold water; disinfecting; flushing with cold water; and blow-drying. Cleaning with cold water removes blood residues. Disinfecting of the knife is carried out, for example, with water at about 82°C, but can also be carried out using cold or hot water to which a disinfectant has been added. The subsequent rinsing with cold water cools the knife and prevents the formation of a layer of protein on the knife. In an optional extra cleaning step, the knife is cleaned with cold water, and optionally blow-dried, before each sample reservoir filled with blood is detached, in order even at this stage to remove as much adhering blood as possible.

In order to administer the blood sample data, each sample reservoir filled with blood is provided with a sample reservoir identifying mark which belongs to a slaughter animal identifying mark arranged on or in said slaughter animal from which the blood in the sample reservoir originates, the sample reservoir identifying mark and the slaughter animal identifying mark are each read by means of a suitable reading device, and data resulting from this reading are stored in combination in a data file, such as a computer memory. After analysis of the blood sample, data relating to the analysis results are added to the said data combination. An example of an identifying mark of a slaughter animal is an earmark or an implanted transponder. Other possible identifying marks are letters, numbers, symbols, patterns or the like, or combinations thereof, which are branded or pierced on parts of the body. A sample reservoir may be provided with a bar code or a dot code, or the like. The identifying marks can preferably be read by machine.

Furthermore, it is preferred for an anti-coagulant to be introduced into each sample reservoir before use, so that it is mixed with the blood sample directly when the sample is taken.

According to the invention, the knife for taking a blood sample of a slaughter animal is characterized according to claim 11.

In a preferred embodiment, the duct opens out at the abovementioned other end into a buffer chamber with at least one outlet to the at least one sample reservoir. The buffer chamber is dimensioned in such a way that during the blood sampling it fills up at least in part with blood.

To assist the flow of blood into the buffer chamber, the latter contains at least one opening to atmosphere. During the blood sampling, this opening functions as a vent for the buffer chamber. The abovementioned opening, or another opening to atmosphere, may serve as an overflow opening for determining a maximum blood level in the buffer chamber, so that the volume of blood collected in the buffer chamber during the blood sampling can be accurately predetermined.

In a preferred embodiment, the buffer chamber outlet to a sample reservoir comprises a tube stub and the sample reservoir comprises a sample tube with a closed end and an opposite open end, it being possible to push the tube stub and the sample tube over one another, and the free end of the tube stub being connected in a sealing manner to the sample tube. Such a sealed design of the buffer chamber outlet and the sample reservoir ensures that blood is reliably drawn from the buffer chamber into the sample reservoir during the removal of the sample tube from the tube stub.

Preferably, the knife blade is provided with a recess for accommodating at least part of a duct which is designed as a tube. In an embodiment of this kind, there is no need to produce a special knife, but rather it is possible to base the design on a standard, commercially available knife, and it is merely necessary to form a recess in the blade of such a knife.

Advantageously, the duct is arranged detachably in the recess, so that the duct and the connected buffer chamber and buffer chamber outlet(s) can simply be detached from a knife which has become worn as a result of use and can be arranged in a new knife provided with a recess. In particular, the duct is adhesively bonded by means of a soluble or softenable adhesive to projections which protrude into the recess. On the other hand, it is possible, to allow quick and easy exchange of knives, to attach the buffer chamber to the blade using a screw or circlip fastening. In this case, the duct running along the blade is stabilized at its free end by providing it with a lip which projects into an associated opening in the blade.

It is also pointed out that the buffer chamber outlets of the knife prevent the hand of the sticker sliding off the handle of the knife and over the blade, so that undesirable injuries are prevented.

In order to achieve as high a level of mechanisation of the blood sampling as possible, the knife is provided with at least one projection for positioning the knife in a knife support which is at least partially of complementary form to the at least one projection. The at least one projection makes it possible to place the knife in the knife support in an unambiguous manner, after which it is possible for the actions comprising removal of the sample reservoir(s), cleaning of the knife and attachment of (a) new sample reservoirs) to take place completely mechanically and automatically.

The knife is preferably provided with two projections which protrude in opposite directions, and part of each projection preferably has a substantially circular cross-section, another part of the projection having a substantially rectangular cross-section. This measure increases the stability of the knife in the knife support.

Furthermore, for the benefit of mechanical and automatic execution of the actions described above, the invention provides a blood sample processing device for processing a blood sample of a slaughter animal, comprising: a knife-holding station for holding a knife according to the invention; a sample reservoir removal station for removing the sample reservoir(s) from the knife; at least one cleaning and/or disinfection station for cleaning and disinfecting the knife; a sample reservoir attachment station for attaching the sample reservoir(s) to the knife; a knife delivery station for delivering the knife; and knife transportation means for transporting the knife past the stations.

In a preferred embodiment, the blood sample processing device comprises sample reservoir transportation means which comprise a clamping device, which is arranged on a movable arm for gripping at least one sample reservoir, as well as a rotatable disc, which is provided with openings, for holding the sample reservoir(s) and attaching/removing small caps thereto/ therefrom, and at least one hose or tube in which the sample reservoir(s) can be transported at superatmospheric pressure or subatmospheric pressure, the arm with clamping device transporting the sample reservoir(s) from the sample reservoir removal station to the disc, and the disc conveying the sample reservoir(s) from the arm with clamping device to the at least one hose or tube, or alternatively the transportation taking place in the opposite direction, from the at least one hose or tube to the sample reservoir attachment station. If one ensures that the small cap of the sample reservoir is unable to pass through the opening while the sample reservoir can pass thorough the opening, the sample reservoir can hang from the small cap in an opening in the disk when the latter is disposed horizontally. By pulling the sample reservoir downwards beneath the disk, the small cap is separated from the reservoir. If the small cap is supported at the top side of the disc, the sample reservoir can easily be pressed firmly onto the small cap through the opening, from the underside of the disk.

Preferably, the knife-holding station comprises a movable knife support for supporting the knife for the purpose of feeding the knife to the knife transportation means in a predetermined position. After use, the sticker places his/her knife in the knife support, which determines the position of the knife and consequently allows the knife to be manipulated mechanically and automatically in the blood sample processing device.

In an advantageous embodiment, the at least one cleaning and disinfection station comprises: a first station with a cold water feed device for cleaning the knife with cold water; a second station with a disinfectant feed device for disinfecting the knife, which disinfectant consists, for example, of water at about 82 °C or of a lactic acid solution or chlorine solution; a third station with a flushing device for rinsing the knife with cold water; and a fourth station with an air-blowing device for blow-drying the knife using air. Preferably, the first station and the second station are each designed for the internal and external cleaning of the knife.

The knife delivery station works in the opposite way to the knife-holding station.

In a preferred embodiment, the knife transportation means comprise a gripper device which engages on the knife for fixing the knife in a predetermined position during transportation of the knife past the stations, which gripper device can be moved between a closed position and an open position. The gripper device allows simple transfer of the knife from the movable knife support (in the knife-holding station) or to the movable knife support (in the knife delivery station).

In a particularly compact and efficient embodiment of the blood sample processing device, the knife transportation means execute a circular movement, and the stations are disposed at predetermined angular positions along the circle.

The invention is explained in more detail with reference to the drawing, in which:
Fig. 1 shows a diagrammatic, perspective view of a device according to the invention;
Fig. 2 shows a top view of a knife according to the invention;
Fig. 2a shows a top view of another knife according to the invention;
Fig. 2b shows a top view of yet another knife according to the invention;
Fig. 3 shows a side view of the knife shown in Fig. 2;
Fig. 3a shows a side view of the knife shown in Fig. 2a;
Fig. 3b shows a side view of the knife shown in Fig. 2b;
Fig. 4 shows a front view of the knife of Fig. 2 in the direction of arrow IV;
Figs. 5a and 5b show a cross-sectional view of the action of sample reservoir(s) coupled to the knife;
Fig. 5c shows a view of the cross-section of the knife on line Vc-Vc in Fig. 3a;
Fig. 5d shows a partial longitudinal section through the knife on line Vd-Vd in Fig. 2a;
Fig. 5e shows a view of the cross-section of the knife on line Ve-Ve in Fig. 3b;
Fig. 5f shows a partial longitudinal section through the knife on line Vf-Vf in Fig. 2b;
Fig. 6 shows, partially in cross-section and partially in side view, a turret device according to the invention;
Fig. 7 shows a diagrammatic plan view of the device in accordance with Fig. 6;
Fig. 8 shows a detail of the device shown in Fig. 6;
Fig. 9 shows a perspective view of a detail of the device shown in Fig. 6;
Fig. 10 shows a perspective view of another detail of the device shown in Fig. 6;
Fig. 11 shows a flow diagram for the purpose of explaining the actions carried out in the device in accordance with Fig. 6; and
Fig. 12 shows the layout of the device in accordance with Fig. 6 in a slaughterhouse.

In the various figures, equivalent reference numerals relate to equivalent components or components which have an equivalent function.

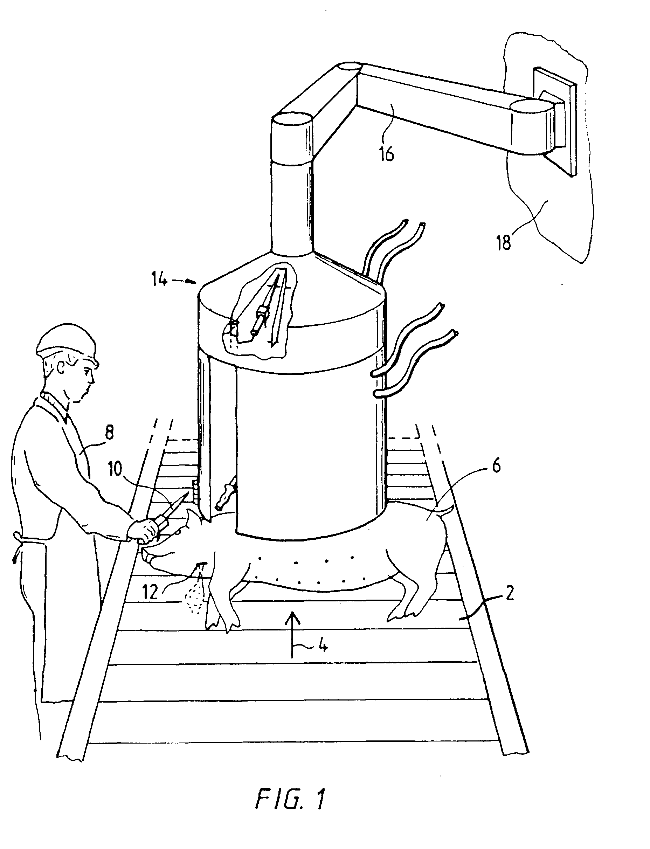

Fig. 1 shows a section of a slaughterhouse, in which stunned pigs 6 lying on their sides are transported to a sticking station on a conveyor belt 2, which advances in the direction of arrow 4. In Fig. 1, only one pig 6 is shown; in practice, pigs 6 lie at such a short distance apart on the conveyor belt 2 that another pig 6 arrives at the sticking station every four seconds. In the sticking station, there is a sticker 8 holding a knife 10 for sticking the pig 6 in the carotid artery. The pig 6 bleeds to death through the cut 12 created in this way.

A turret device 14 is suspended above the conveyor belt 2 and above the pigs 6, within easy reach of the sticker 8. For sticking each pig 6, the sticker 8 takes a knife out of the turret device 14, sticks the pig and places the knife 10 back in the turret device 14. The structure of the knife 10 and the turret device 14 will be explained in more detail below. The turret device 14 is suspended from a wall 18 of the slaughterhouse by means of a pivot arm 16, so that the sticker 8 can select the position of the turret device 14 in such a manner that knives can be taken out of and replaced in the turret device 14 as easily and quickly as possible.

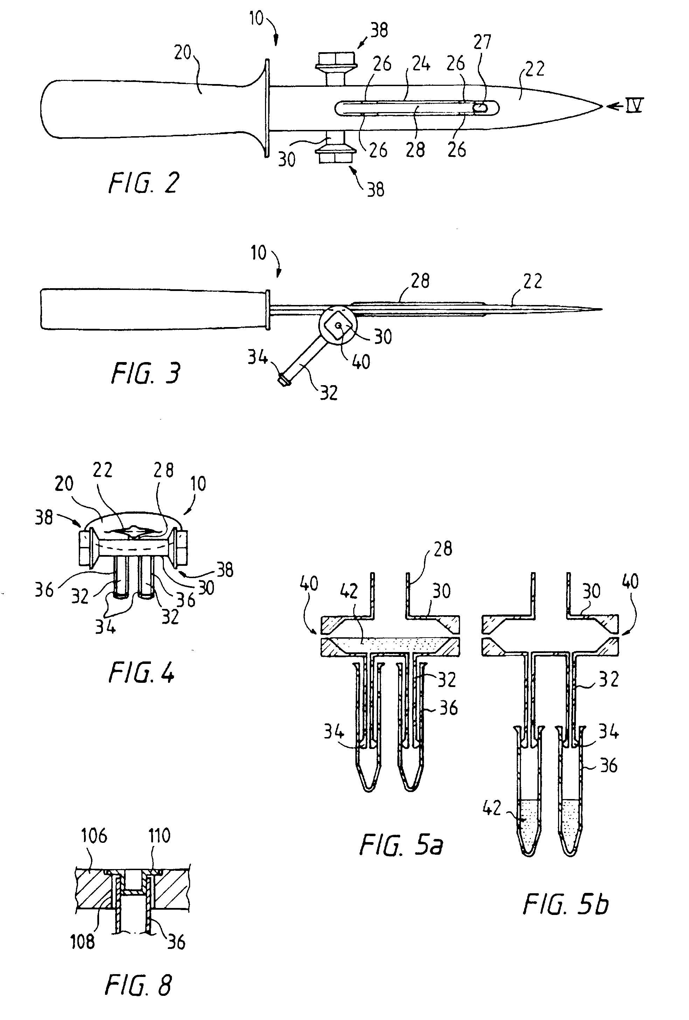

Figs. 2-4 show the knife 10 in more detail. The knife 10 comprises a handle 20 and a blade 22, in which there is formed a recess 24 which extends in the longitudinal direction of the blade. Four projections 26 protrude inwards into the recess 24. Between the projections 26 there is detachably arranged, for example by snap-fitting or adhesive bonding, a section of a tube 28 which is open on the side of the point of the blade 22. The tube 28 is coupled to and in liquid communication with a buffer chamber 30, which in turn is coupled to and in liquid communication with two buffer chamber outlets 32, which are in the form of tube stubs and have an annular seal 34 at their free end. Sample reservoirs 36 made of a transparent material are pushed around the buffer chamber outlets 32. The seals 34 hold the sample reservoirs 36 fixed to the buffer chamber outlets 32. The buffer chamber 30, which in the embodiment shown is substantially cylindrical, tapers conically on the outside towards both ends and ends in the form of a rectangle with a square cross-section. The partially conical, partially rectangular projections 38 are each provided with an opening 40, via which the interior of the buffer chamber 30 is in open communication with the atmosphere.

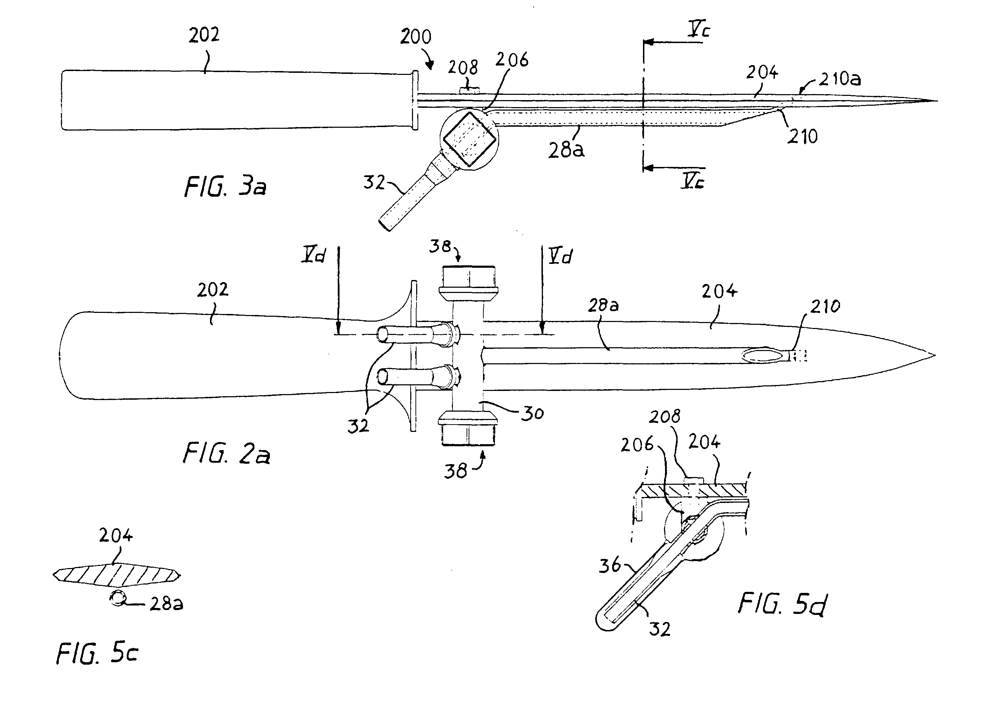

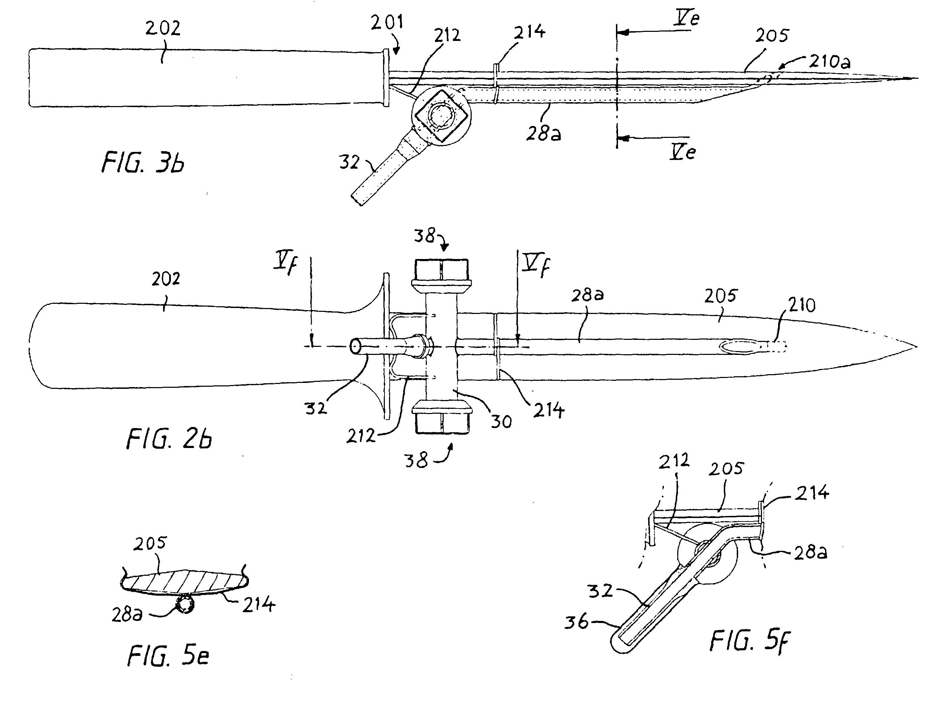

Figs. 5a and 5b diagrammatically clarify the function of the buffer chamber 30 and the sample reservoirs 36. When the blade 22 of the knife 10 is stuck into a blood vessel of an animal to be slaughtered, blood flows via the opening 27, through the tube 28 and into the buffer chamber 30, the openings 40 acting as vent and overflow openings. A level of blood 42 which is shown in Fig. 5a is thus established in the buffer chamber 30. If the sample reservoirs 36 are then moved away from the buffer chambers outlets 32, the seal 34 ensures that the blood 42 from the buffer chamber 30 is drawn into the sample reservoirs 36, the openings 40 serving the purpose of aeration, as illustrated in Fig. 5b. The quantity of blood 42 in the buffer chamber 30 as shown in Fig. 5a is distributed evenly between the two sample reservoirs 36, as shown in Fig. 5b.

Figs. 2a, 3a, 5c and 5d show a knife 200 with a handle 202 and a blade 204. On one side of the blade 204, a hollow tube 28a is arranged substantially parallel to the longitudinal direction of the blade 204. In accordance with the design shown in Fig. 2, 3 and 4, the tube 28a in Figs. 2a, 3a, 5c and 5d is connected to a buffer chamber 30 which is provided with partially frustoconical and partially rectangular projections 38. Two buffer chamber outlets 32 can be provided with sample reservoirs 36, as shown in particular in Fig. 5d. The buffer chamber 30 is provided with a support 206 with a hole which is provided with an internal screw thread. At the location where the support 206 touches the blade 204, the blade 204 is provided with a through-hole. The support 206 can thus be attached to the blade 204 by means of a screw 208, with the result that the tube 28a, the buffer chamber 30 with projections 38 and the buffer chamber outlets 32 are fixed in position with respect to the blade 204. In order to prevent deformation of the tube 28a, the latter is provided at its free end with a lip 210 which is accommodated in a hole 210a in the blade 204.

Figs. 2b, 3b, 5e and 5f show a knife 201 with a handle 202 and a blade 205. The knife 201 differs only slightly from the knife 200 which is shown in Fig. 2a, 3a, 5c and 5d. Instead of the support 206, which is attached to the blade 204 using a bolt 208, of the knife 200, the knife 201 is provided with two circlips 212 and 214, which interact with the lip 210 so as to fix the blood sampling section of the knife 201 in position with respect to the blade 205. As shown in particular by Fig. 5e, the circlip 214 clamps around the blade 205, while the spring 212 ensures that the lip 210 is fixed in the opening 210a.

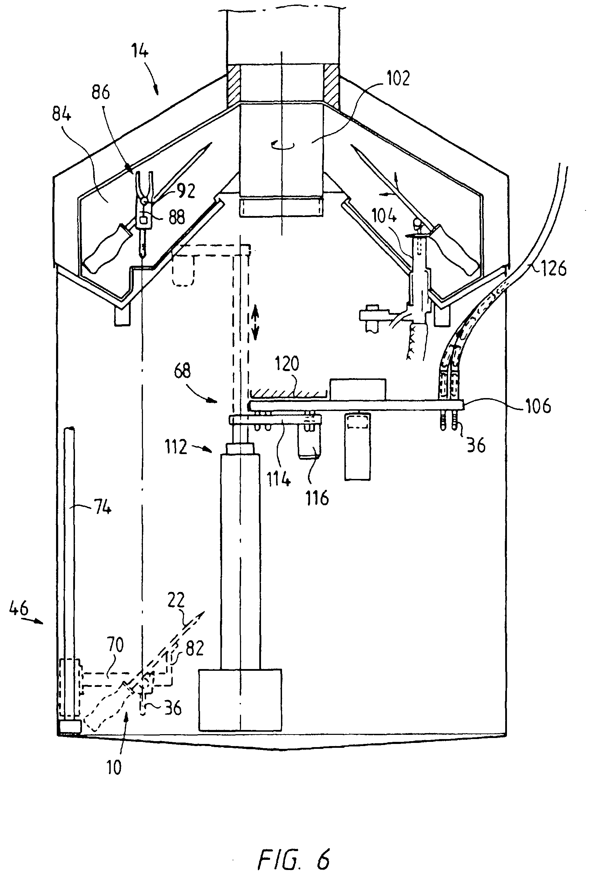

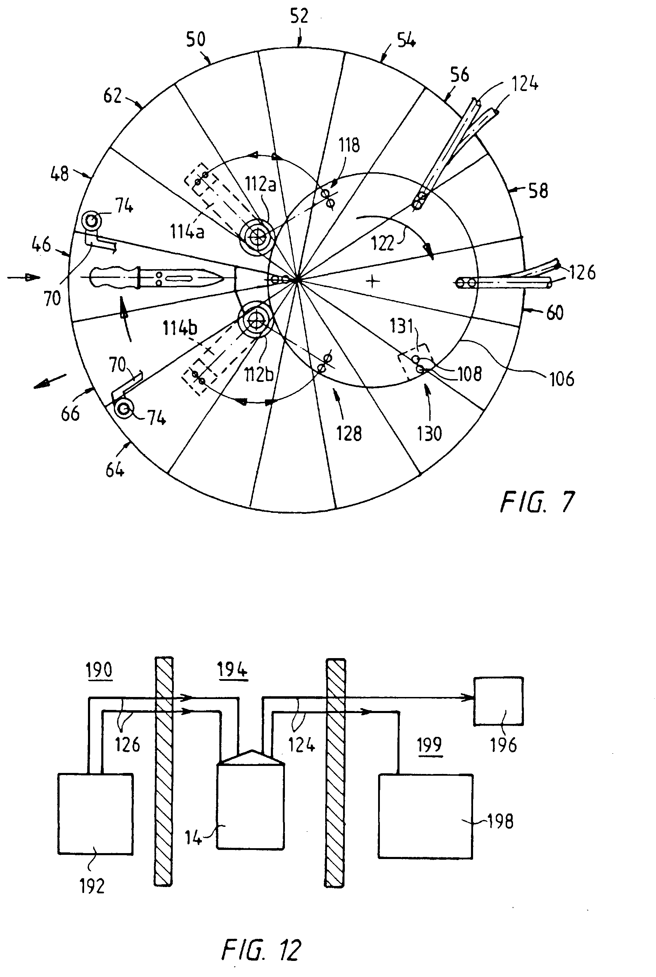

Figs. 6 and 7 show the turret device 14 in which the knife 10 can be placed after sticking, and in which the sample reservoirs 36 are removed from the knife, sealed and removed, and the knife 10 is cleaned, disinfected and provided with new sample reservoirs 36, after which the knife is presented again to the sticker.

For this purpose, the turret device 14 comprises a knife-holding station 46, a number of cleaning and disinfection stations 48, 50, 52, 54, 56, 58 and 60, a sample reservoir removal station 62, a sample reservoir attachment station 64, a knife delivery station 66, and transportation means for transporting the knife past the various stations. Furthermore, the turret device 14 comprises sample reservoir transportation means 68 for removing and feeding blood-filled and empty sample reservoirs.

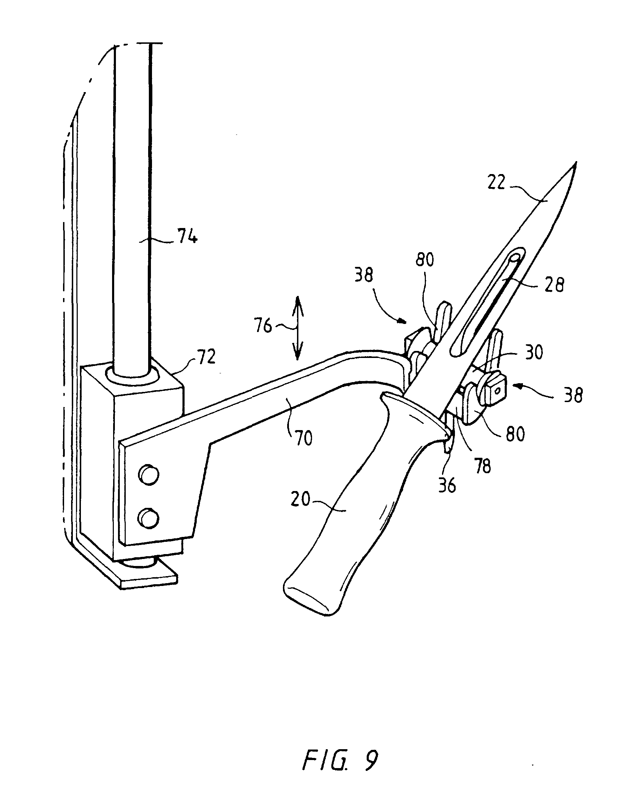

The knife-holding station 46 will be described in more detail with reference to Figs. 6 and 9. The holding station 46 comprises an arm 70 which is attached to a so-called piston-rod-free cylinder 72, and as a result can be moved along a rod 74 in the directions of double arrow 76. At the end remote from the cylinder 72, the arm 70 bears a support device 78 which comprises two holding forks 80 with teeth of unequal length, the holding forks 80 being shaped and arranged in such a manner that the buffer chamber 30 can be held between their teeth. As shown in Fig. 6, it is additionally possible to provide a support 82 for supporting the blade 22 of the knife 10.

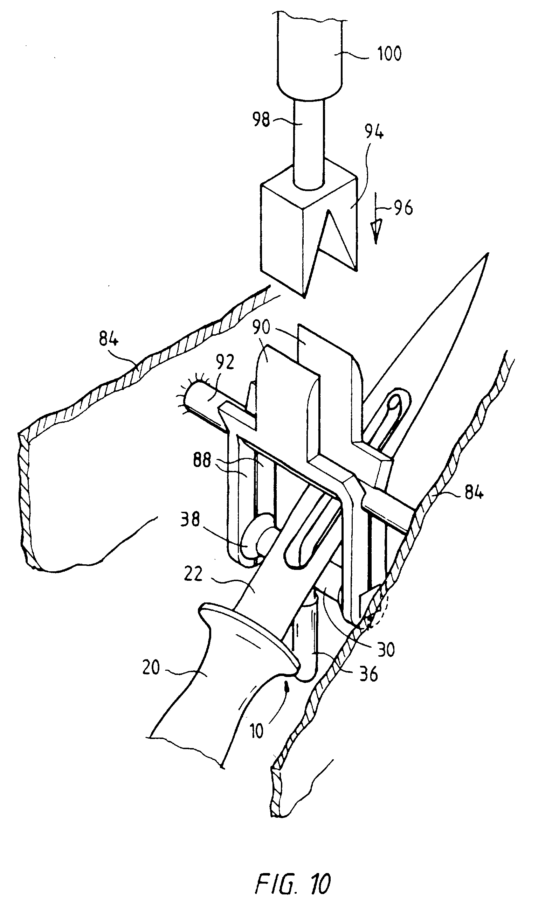

As illustrated in more detail in Figs. 6 and 10, in the holding station 46 the knife 10 is transported upwards towards a clamping device 86 which is disposed between plates 84. The clamping device 86 is attached between the plates 84 and comprises two clamping parts 88, the bottom ends of which can be moved apart in the manner of a clothes peg and are provided with rectangular recesses for accommodating the rectangular part of the projections 38 on the buffer chamber 30 in a substantially form-fitting manner. By moving the protrusions 90 of the clamping parts 88 towards one another, the clamping parts 88 pivot, under the opposing force of a spring (not shown in more detail), around rod 92, during the course of which pivoting the projections 38 of the knife 10 can be accommodated but also released again. The protrusions 90 can be moved towards one another, for example, by moving a block 94 with a V-shaped recess over the protrusions 90, in the direction of arrow 96. The block 94 can be driven in a manner which is not shown in more detail by means of a piston rod 98 of a piston-cylinder unit 100. In the clamping device 86, between the plates 84, the knife is moved through the abovementioned stations by means of a displacement of this unit through predetermined angles by means of an indexed movement of the plates 84 and the clamping device 86 using a vertical shaft 102 to which the plates 84 are attached. The shaft 102 is provided along its entire circumference with plates 84 which are arranged at regular angular distances and have clamping devices 86 arranged between them, so that knives 10 can be received continuously, can be guided in steps through the various stations and can be delivered again. The turret device 14 depicted in Fig. 6 and 7 contains sixteen plates 84 and the same number of clamping devices 86.

The knife delivery station 66 in principle contains the same components as the knife-holding station 46, but works in the opposite way: by opening the clamping parts 88, the knife 10 which is held in the clamping device 86 is lowered into the fork parts 80 of the support device 78, which for this purpose has been placed directly beneath the clamping device 86. Then, the arm 70 together with the knife 10 in the support device 78 is moved downwards along the rod 74, after which the sticker can take the knife 10 out of the holding forks 80.

In the cleaning station 48, the outside of the knife 10 is sprayed with cold water for the purpose of removing blood residues. Details of spraying means are not shown here, since the person skilled in the art can design these based on his/her own experience. The knife 10 is then blow-dried in a manner not shown in more detail in the cleaning station 48 with the aid of compressed air. In the sample reservoir removal station 62, the sample reservoirs are removed from the knife 10 in a manner which will be discussed in more detail below. The inside of the knife 10 is then cleaned in the cleaning station 50 using cold water, for example by spraying water, via a suitable nozzle 104 which is placed mechanically on the buffer chamber outlets, through the buffer chamber outlets 32, the buffer chamber 30 and the tube 28. This is illustrated diagrammatically in Fig. 6 in an angular position which, for the sake of clarity, differs from the angular position shown for the cleaning station 50 in Fig. 7. In the cleaning station 52, the outside of the knife 10 is then cleaned with cold water in a manner which is not shown in more detail. The inside of the knife is then disinfected in the disinfection station 54 using hot water at a temperature of about 82°C, for example with the aid of a similar nozzle to the nozzle 104 which is shown in Fig. 6. The outside of the knife 10 is then disinfected in the disinfection station 56 using hot water at a temperature of about 82°C. Finally, in a manner which is not shown in more detail, the knife 10 is rinsed with cold water in the cleaning station 58 and is blow-dried with compressed air in the cleaning station 60. When the knife 10 has arrived in the sample reservoir attachment station 64, new sample reservoirs are attached to the buffer chamber outlets 32 in a manner which will be discussed in more detail below.

In the turret device 14, there are arranged the sample reservoir transportation means 68, which comprise an indexed disc 106 which can be rotated in a horizontal plane through predetermined angles and in which sets of openings 108, the pitch of which in the radial direction corresponds to the pitch of the buffer chamber outlets 32 of the knife 10, are arranged at angular distances of 60°. The openings 108 are shown in more detail in Fig. 8, which also shows a sample reservoir 36 which is provided with a cap 110. The sample reservoir 36 is suspended from the cap 110 in the opening 108 which has two different diameters. In the turret device 14, transverse arms 114a and 114b are arranged on two telescopically extendable arms 112a and 112b, which transverse arms 114a and 114b can pivot about the telescopic arms and are each provided at their free ends with a clamping device 116, which is not shown in more detail and in which two sample reservoirs 36 can be clamped.

As shown diagrammatically in Figs. 6 and 7, when the knife is situated in the sample reservoir removal station 62, the arm 112a is extended to a suitable length and the transverse arm 114a is moved into the position indicated by dashed lines in Fig. 7. The sample reservoirs 36 situated on the knife 10 are then gripped by the associated clamping device 116 and are pulled off the buffer chamber outlets 32 by retracting the arm 112a. The transverse arm 114a can then be pivoted beneath the disc 106, the blood-filled sample reservoirs moving into the position indicated in Fig. 7 by 118. Those ends of the sample reservoirs 36 which protrude above the transverse arm 114a and the clamping device 116 are then moved into the associated openings 108 in the disc 106 by extending the arm 112a, during which operation the sample reservoirs 36 are simultaneously sealed by a cap 110 which is already situated in the opening 108 and is held in position 118 above the disc 106 by means of a stop 120. After the disc 106 has been rotated through 60° in the direction of arrow 122, the blood-filled and sealed sample reservoirs 36 are drawn into hoses 124 and removed with the aid of subatmospheric pressure. When the disc 106 has again been rotated through 60° in the direction 122, new, empty sample reservoirs, which are provided with small caps, are placed in the vacant openings 108 by supplying these reservoirs through hoses 126 with the aid of compressed air. After being rotated through a further 120°, the new sample reservoirs are gripped by the clamping device 116, which is arranged on the transverse arm 114b, at the location indicated by 128 and, by means of sliding movements of the arm 112b and a pivoting movement of transverse arm 114b, they are moved towards and arranged on a cleaned knife 10. The small caps 110 of the empty sample reservoirs 36 remain behind at position 128, and these small caps 110 are used in the position of the disc 106 which is indicated by 118 to close off other blood-filled sample reservoirs 36.

On the side remote from the sample tube 36, the small cap 110 may be provided with a machine-readable code, which, for example in the position of the disc 106 indicated by 130, can be read with the aid of a sensor 131 which is arranged above the disc 106 and is merely indicated by dashed lines.

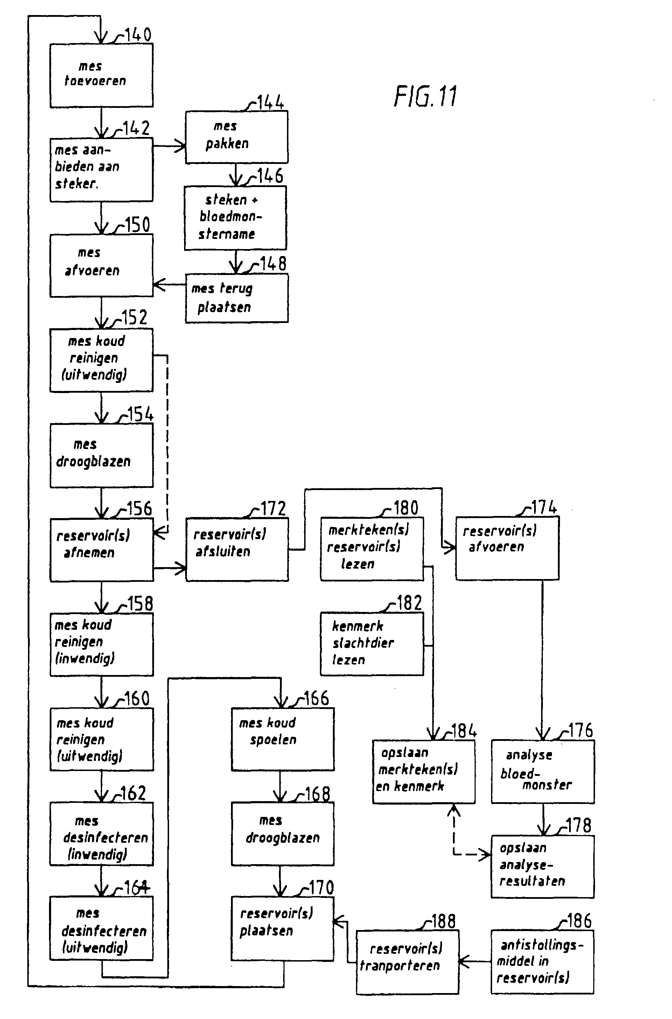

Fig. 11 shows the actions carried out in the turret device 14 and other actions associated with the blood sampling, in the logical order indicated by arrows. In step 140, the knife 10 is moved to the knife delivery station 66. In the knife delivery station 66, the knife is then presented to the sticker, as indicated by step 142. In step 144, the sticker takes hold of the knife, after which, in step 146, the animal for slaughtering is stuck and a blood sample is left in the knife. In step 148, the sticker then places the knife 10 back in the turret device 14, into the knife-holding station 46. In step 150, the knife 10 is discharged from the knife-holding station 46 into the cleaning station 48. If, after step 142, the sticker does not take hold of the knife, the unused knife is removed in step 150. In step 152, the outside of the knife is cleaned with cold water in the cleaning station 48 and then, in step 154, it is blow-dried using compressed air. If appropriate, step 154 can be missed out, as indicated by a dashed line. Then, in step 156, which is carried out in the sample reservoir removal station 62, the sample reservoirs 36 are removed from the knife. The inside of the remaining part of the knife is cleaned with cold water in step 158 in cleaning station 50, and then the outside is cleaned with cold water in step 160 in cleaning station 52, after which, in step 162, the inside is disinfected in disinfection station 54, then the outside is disinfected in step 164 in disinfection station 56, then in step 166 the knife is rinsed with cold water in cleaning station 58 and finally blow-dried in step 168 using compressed air in cleaning station 60. Then, in step 170, new sample reservoirs are placed on the knife in sample reservoir attachment stations 64. The knife is now ready to be used again, so that step 140 can then follow. After step 156, comprising the removal of the reservoirs from the knife, in step 172 the reservoirs are sealed at the location indicated in Fig. 7 by 118. The reservoirs are subsequently discharged in step 174, one sample reservoir being discharged to an analysis device and the other sample reservoir being temporarily stored. In step 176, the said one blood sample is analyzed, after which, in step 178, the analysis results are stored in a data file. In addition, in step 180, an identifying mark provided on the sample reservoirs is read and in step 182 a classification mark of the slaughter animal is read. In step 184, data relating to the identifying mark and the classification mark are stored in a data file. This data is coupled with the results stored in step 178, as indicated by the dashed line between steps 184 and 178.

Prior to step 170, which comprises placing the sample reservoirs on the knife, in a step 186 an anti-coagulant is placed in each sample reservoir, and in step 188 the sample reservoirs are transported towards the turret device 14, in particular the rotatable disc 106.

Fig. 12 illustrates the position of various components of the sampling system according to the invention. A sample reservoir feed device 192 is disposed in a room 190, from where new, empty sample reservoirs, which are provided with a cap, are fed via the hoses 126 to the turret device 14. The turret device is situated in a room 194 which is separated from the room 190. From the turret device 14, blood-filled sample reservoirs 36 which are provided with a cap are discharged, via the hoses 124, to a storage device 196 and an analysis device 198. The latter two devices 196 and 198 are situated in a room 196 which is separate from the room 194. The rooms 190 and 196 may if desired coincide. Separating the room 194 from the rooms 190 and 196 assists with the hygiene of the blood sampling.

## Claims

1. Method for processing blood of an individual slaughter animal, comprising the steps of:
cutting a blood vessel of the slaughter animal so as to release blood;
taking a blood sample (42) from the blood released;
analyzing the blood sample,
characterized in that the blood sample (42) is taken during sticking of the slaughter animal (6) in a slaughterhouse at the sticking place (12) in the slaughter animal, and that the blood sample (42) is collected in at least one sample reservoir (36) which is coupled to, and movable with a knife (10) used for the sticking, whereafter the at least one sample reservoir (36) is detached from the knife (10) for analyzing the blood sample.

2. Method according to claim 1, characterized in that the blood sample (42) is collected in two different sample reservoirs (36) which are coupled to the knife (10).

3. Method according to claim 1 or 2, characterized in that after detaching each blood-filled sample reservoir (36) from the knife (10), the knife (10) is cleaned and disinfected, and then (a) new sample reservoir(s) (36) is/are coupled to the knife.

4. Method according to claim 3, characterized in that one sample reservoir (36), which has been filled with blood during the sticking of the slaughter animal (6), is sealed after it has been detached from the knife (10) and is conveyed to a room (196) which is situated in the slaughterhouse and where one or more predetermined analyses are carried out on the blood sample (42).

5. Method according to claim 4, characterized in that the sample reservoir (36) is conveyed in a hose (124) or tube with the aid of a fluid which is at superatmospheric or subatmospheric pressure.

6. Method according to any of claims 3 - 5, characterized in that the cleaning and disinfection of the knife (10) after each sample reservoir (36) filled with blood (42) has been detached comprises the following steps:
- cleaning with cold water;
- disinfecting, in particular with water at 82 °C;
- flushing with cold water; and
- blow-drying.

7. Method according to claim 6, characterized in that the knife (10) is cleaned with cold water, and optionally blow-dried, before each sample reservoir (36) filled with blood (42) is detached.

8. Method according to any of claims 1 - 7, characterized in that each sample reservoir (36) filled with blood (42) is provided with a sample reservoir identifying mark which belongs to a slaughter animal identifying mark arranged on or in said slaughter animal (6) from which the blood in the sample reservoir originates, the sample reservoir identifying mark and the slaughter animal identifying mark are each read using a reading device (131), and data resulting from this reading are stored in combination in a data file.

9. Method according to claim 8, characterized in that, after analysis of the blood sample (42), data relating to the analysis results are added to the said data combination.

10. Method according to any of claims 1 - 9, characterized in that an anti-coagulant is introduced into each sample reservoir (36) prior to use.

11. Knife provided with a duct (28) with an open end (27) in the region of the knife blade (22) which is intended to penetrate into a slaughter animal (6) for sticking the slaughter animal, which duct (28) is in liquid communication, at the other end, with at least one sample reservoir (36) which is coupled to, and movable with the knife, the at least one sample reservoir (36) being detachably connected to the knife (10) and being adapted to contain the blood of an individual slaughter animal.

12. Knife according to claim 11, characterized in that the duct (28) opens out at said other end into a buffer chamber (30) with at least one outlet to the at least one sample reservoir (36).

13. Knife according to claim 12, characterized in that the buffer chamber (30) is provided with at least one opening (40) to atmosphere for venting and aerating the buffer chamber.

14. Knife according to claim 12 or 13, characterized in that the buffer chamber (30) is provided with at least one opening (40) to atmosphere for determining a maximum blood level in the buffer chamber.

15. Knife according to any of claims 12 - 14, characterized in that the buffer chamber outlet comprises a tube stub (32) and in that the sample reservoir (36) comprises a sample tube with a closed end and an opposite open end, it being possible to push the tube stub and the sample tube over one another, and the free end of the tube stub being connected in a sealing manner (34) to the sample tube.

16. Knife according to any of claims 11 - 15, characterized in that the knife blade (22) is provided with a recess (24) for accommodating at least part of a duct which is designed as a tube (28).

17. Knife according to claim 16, characterized in that the duct (28) is arranged detachably in the recess (24).

18. Knife according to claim 17, characterized in that the duct (28) is adhesively bonded to projections (26) which protrude into the recess (24).

19. Knife according to any of claims 11 - 15, characterized in that the knife blade (204; 205) is provided with a hole (210) for accommodating one end of the duct (28a).

20. Knife according to any of claims 11 - 15 or 19, characterized in that the buffer chamber (30) is attached to the knife blade (204; 205).

21. Knife according to claim 20, characterized in that the attachment is formed by a screw fastening.

22. Knife according to claim 20, characterized in that the attachment is formed by a circlip fastening (212, 214).

23. Knife according to any of claims 11 - 22, characterized by at least one projection (38) for positioning the knife (10) in a knife support (80; 88) which is of at least partially complementary form to the at least one projection.

24. Knife according to claim 23, characterized by two projections (38) which protrude in opposite directions.

25. Knife according to claim 23 or 24, characterized in that part of each projection (38) has a substantially circular cross-section, and in that another part of the projection has a substantially rectangular cross-section.

26. Blood sample processing device (14) for processing a blood sample (42) of a slaughter animal (6), comprising:
- a knife-holding station (46) for holding a knife (10) according to any of claims 11 - 25;
- a sample reservoir removal station (62) for removing the sample reservoir(s) (36) from the knife (10);
- at least one cleaning and/or disinfection station (48, 50, 52, 54, 56, 58, 60) for cleaning and disinfecting the knife (10);
- a sample reservoir attachment station (64) for attaching the sample reservoir(s) (36) to the knife (10);
- a knife delivery station (66) for delivering the knife (10); and
- knife transportation means for transporting the knife (10) past the stations.

27. Blood sample processing device according to claim 26, characterized by sample reservoir transportation means which comprise a clamping device (116), which is arranged on a movable arm (112, 114) for gripping at least one sample reservoir (36), as well as a rotatable disc (106), which is provided with openings, for holding the sample reservoir(s) (36) and attaching/removing small caps (110) thereto/therefrom, and at least one hose (124, 126) or tube in which the sample reservoir(s) (36) can be transported at superatmospheric pressure or subatmospheric pressure, the arm (112, 114) with clamping device (116) transporting the sample reservoir(s) (36) from the sample reservoir removal station (62) to the disc (106), and the disc (106) conveying the sample reservoir(s) (36) from the arm (112, 114) with clamping device (116) to the at least one hose (124) or tube, or alternatively the transportation taking place in the opposite direction, from the at least one hose (126) or tube to the sample reservoir attachment station (64).

28. Blood sample processing device according to claim 27, characterized in that the small cap (110) has dimensions which are such that it is unable to pass through the openings (108) in the disk (106), while the sample reservoir (36) has dimensions which are such that it can pass through the openings (108) in the disk (106).

29. Blood sample processing device according to any of claims 26 - 28, characterized in that the knife-holding station (46) comprises a movable knife support (70, 72, 74, 78, 80) for supporting the knife (10) for the purpose of feeding the knife (10) to the knife transportation means in a predetermined position.

30. Blood sample processing device according to any of claims 26 - 29, characterized in that the at least one cleaning and disinfection station comprises:
- a first station (50, 52) with a cold water feed device for cleaning the knife 910) with cold water;
- a second station (54, 56) with a disinfectant feed device for disinfecting the knife (10);
- a third station (58) with a flushing device for rinsing the knife (10) with cold water; and
- a fourth station (60) with an air-blowing device for blow-drying the knife (10) using air.

31. Blood sample processing device according to claim 30, characterized in that the first station (50, 52) and the second station (54, 56) are each designed for the internal and external cleaning/disinfection of the knife (10).

32. Blood sample processing device according to any of claims 26 - 31, characterized in that the knife delivery station (66) comprises a movable knife support (70, 72, 74, 78, 80) for supporting the knife (10) for the purpose of removing the knife (10) from the knife transportation means in a predetermined position.

33. Blood sample processing device according to any of claims 26 - 32, characterized in that the knife transportation means comprise a gripper device (86) which engages on the knife (10) for fixing the knife (10) in a predetermined position during transportation of the knife (10) past the stations, which gripper device (86) can be moved between a closed position and an open position.

34. Blood sample processing device according to any of claims 26 - 33, characterized in that the knife transportation means execute a circular movement, and in that the stations are disposed at predetermined angular positions along the circle.

## Patentansprüche

1. Verfahren zur Behandlung von Blut eines einzelnen Schlachttieres, das die folgenden Schritte aufweist:
Aufschneiden eines Blutgefäßes des Schlachttieres, um Blut abzulassen,
Entnahme einer Blutprobe (42) des abgelassenen Bluts, Analyse der Blutprobe,
dadurch gekennzeichnet, daß die Blutprobe (42) beim Abstechen des Schlachttieres (6) in einem Schlachthof an der Stichstelle (12) des Schlachttieres entnommen wird, und daß die Blutprobe (42) in mindestens einem Blutprobenbehälter (36) gesammelt wird, der mit einem zum Abstechen verwendeten Messer verbunden und beweglich ist, wonach der mindestens eine Blutprobenbehälter (36) zur Analyse der Blutprobe vom Messer (10) abgenommen wird.

2. Verfahren nach Anspruch 1,
dadurch gekennzeichnet, daß die Blutprobe (42) in zwei verschiedenen Blutprobenbehältern (36) gesammelt wird, die mit dem Messer (10) verbunden sind.

3. Verfahren nach Anspruch 1 oder 2,
dadurch gekennzeichnet, daß das Messer (10) nach Abnehmen der mit Blut gefüllte Blutprobenbehälter (36) gereinigt und desinfiziert wird und daß dann (ein) neue(r) Blutprobenbehälter (36) mit dem Messer verbunden (wird) werden.

4. Verfahren nach Anspruch 3,
dadurch gekennzeichnet, daß ein Blutprobenbehälter (36), der während des Abstechens des Schlachttieres (6) mit Blut gefüllt worden ist, versiegelt wird, nachdem er vom Messer (10) abgenommen worden ist und in einen Raum (196) transportiert wird, der sich im Schlachthof befindet und in dem ein oder mehrere vorbestimmte Analysen an der Blutprobe (42) durchgeführt werden.

5. Verfahren nach Anspruch 4,
dadurch gekennzeichnet, daß der Blutprobenbehälter (36) in einem Schlauch (124) oder einem Rohr mit Hilfe eines unter Über- oder Unterdruck stehenden Fluids transportiert wird.

6. Verfahren nach einem der Ansprüche 3 bis 5,
dadurch gekennzeichnet, daß die Reinigung und Desinfektion des Messers (10) nach Abnehmen jedes mit Blut (42) gefüllten Blutprobenbehälters (36) die folgenden Schritte umfaßt:
- Reinigung mit kaltem Wasser;
- Desinfektion, insbesondere mit Wasser bei 82°C;
- Spülen mit kaltem Wasser; und
- Trockenblasen.

7. Verfahren nach Anspruch 6,
dadurch gekennzeichnet, daß das Messer (10) mit kaltem Wasser gereinigt und wahlweise trockengeblasen wird, bevor jeder mit Blut (42) gefüllte Probenbehälter (36) abgenommen wird.

8. Verfahren nach einem der Ansprüche 1 bis 7,
dadurch gekennzeichnet, daß jeder mit Blut (42) gefüllte Probenbehälter (36) mit einer Probenbehälter-Identifikationsmarke versehen wird, die zu einer Schlachttier-Identifikationsmarke gehört, die auf oder in dem Schlachttier (6) angebracht ist, von dem das Blut im Probenbehälter stammt, daß die Probenbehälter-Identifikationsmarke und die Schlachttier-Identifikationsmarke mittels einer Leseeinrichtung (131) gelesen werden, und daß die erfaßten Daten in Kombination in einer Datendatei gespeichert werden.

9. Verfahren nach Anspruch 8,
dadurch gekennzeichnet, daß nach der Analyse der Blutprobe (42) die Daten dieser Analyse zu der genannten Datenkombination hinzugefügt werden.

10. Verfahren nach einem der Ansprüche 1 bis 9,
dadurch gekennzeichnet, daß vor der Benutzung jedem Probenbehälter (36) ein Antigerinnungsmittel zugegeben wird.

11. Messer mit einem Kanal (28) mit einem offenen Ende (27) im Bereich der Messerschneide (22), die zum Abstechen in ein Schlachttier (6) eindringen soll, wobei der Kanal (28) am anderen Ende in Strömungsverbindung mit mindestens einem Probenbehälter (36) steht, der abnehmbar mit dem Messer (10) verbunden und damit bewegbar und der zur Aufnahme von Blut eines einzelnen Schlachttieres ausgebildet ist.

12. Messer nach Anspruch 11,
dadurch gekennzeichnet, daß der Kanal (28) am anderen Ende in eine Aufnahmekammer (30) mit mindestens einem Auslaß zu dem mindestens einen Probenbehälter (36) mündet.

13. Messer nach Anspruch 12,
dadurch gekennzeichnet, daß die Aufnahmekammer (30) mit mindestens einer Öffnung (40) zur Atmosphäre für die Ventilation und die Belüftung des Aufnahmeraumes versehen ist.

14. Messer nach Anspruch 12 oder 13,
dadurch gekennzeichnet, daß die Aufnahmekammer (30) mindestens eine Öffnung (40) zur Atmosphäre aufweist, die zur Bestimmung des maximalen Blutniveaus im Aufnahmeraum dient.

15. Messer nach einem der Ansprüche 12 bis 14,
dadurch gekennzeichnet, daß die Öffnung in der Aufnahmekammer einen Rohrstutzen (32) aufweist, und daß der Probenbehälter (36) ein Rohr für Proben mit einem geschlossenen und einem gegenüberliegenden offenen Ende aufweist, derart, daß der Rohrstutzen und das Rohr für die Proben übereinander schiebbar und das freie Ende des Rohrstutzens gegen das Probenrohr durch eine Dichtung (34) abdichtbar sind.

16. Messer nach einem der Ansprüche 11 bis 15,
dadurch gekennzeichnet, daß die Messerschneide (22) mit einer Ausnehmung (24) zur Aufnahme mindestens eines Teils einer Leitung versehen ist, die als Rohr (28) ausgebildet ist.

17. Messer nach Anspruch 16,
dadurch gekennzeichnet, daß die Leitung (28) abnehmbar in der Ausnehmung (24) angeordnet ist.

18. Messer nach Anspruch 17,
dadurch gekennzeichnet, daß die Leitung (28) haftend mit Vorsprüngen (26) verbunden ist, die in die Ausnehmung (24) ragen.

19. Messer nach einem der Ansprüche 11 bis 15,
dadurch gekennzeichnet, daß die Messerschneide (204; 205) ein Loch (210) zur Aufnahme eines Endes der Leitung (28a) aufweist.

20. Messer nach einem der Ansprüche 11 bis 15 oder 19,
dadurch gekennzeichnet, daß die Aufnahmekammer (30) mit der Messerschneide (204; 205) verbunden ist.

21. Messer nach Anspruch 20,
dadurch gekennzeichnet, daß die Verbindung über eine Schraubverbindung erfolgt.

22. Messer nach Anspruch 20,
dadurch gekennzeichnet, daß die Verbindung durch eine Sprengring-Befestigung (212, 214) erfolgt.

23. Messer nach einem der Ansprüche 11 bis 22,
dadurch gekennzeichnet, daß mindestens ein Vorsprung (38) zur Positionierung des Messers (10) in einem Messerhalter (80; 88) vorgesehen ist, der mindestens teilweise an den mindestens einen Vorsprung angepaßt ist.

24. Messer nach Anspruch 23,
gekennzeichnet durch zwei in entgegengesetzte Richtungen weisende Vorsprünge (38).

25. Messer nach Anspruch 23 oder 24,
dadurch gekennzeichnet, daß ein Teil jedes Vorsprungs (38) einen im wesentlichen runden Querschnitt und ein anderer Teil des Vorsprungs einen im wesentlichen rechteckigen Querschnitt hat.

26. Blutprobenbehandlungsvorrichtung (14) zur Behandlung einer Blutprobe (42) eines Schlachttieres (6), umfassend:
- eine Messerhaltestation (46) zum Halten eines Messers (10) nach einem der Ansprüche 11 bis 25;
- eine Probenbehälterentnahmestation (62) zum Entfernen des (der) Probenbehälter(s) (36) vom Messer (10);
- mindestens eine Reinigungs- und/oder Desinfektionsstation (48, 50, 52, 54, 56, 58, 60) zum Reinigen und Desinfizieren des Messers (10);
- eine Probenbehälteranbringstation (64) zum Anbringen des (der) Probenbehälter(s) (36) am Messer (10);
- eine Messerausgabestation (66) zur Ausgabe des Messers (10); und
- Messertransportmittel zum Transport des Messers (10) durch die Stationen.

27. Blutprobenbehandlungsvorrichtung nach Anspruch 26, gekennzeichnet durch Probenbehältertransportmittel, die eine Klemmvorrichtung (116), die auf einem beweglichen Arm (112, 114) angeordnet ist, um mindestens einen Probenbehälter (36) zu greifen, und eine drehbare Scheibe (106) aufweisen, die mit Öffnungen zum Halten des (der) Probenbehälter(s) und Anbringen/Abnehmen schmaler Kappen (110) am/vom Probenbehälter, und mindestens einen Schlauch (124, 126) oder ein Rohr, in dem der (die) Probenbehälter bei Über- oder Unterdruck transportierbar ist (sind), wobei der Arm (112, 114) mit der Klemmvorrichtung (116) den (die) Probenbehälter von der Probenbehälterentnahmestation (62) zur Scheibe (106) transportiert, die den (die) Probenbehälter (36) vom Arm (112, 114) mit der Klemmvorrichtung (116) zu dem mindestens einen Schlauch (124) oder Rohr transportiert oder alternativ in umgekehrter Richtung von dem mindestens einen Schlauch (126) oder Rohr zur Probenlagerentnahmestation (64).

28. Blutprobenbehandlungsvorrichtung nach Anspruch 27,
dadurch gekennzeichnet, daß die schmale Kappe (110) solche Abmessungen hat, daß sie nicht durch die Öffnungen (108) in der Scheibe (106) paßt, während der Probenbehälter (36) solche Abmessungen hat, daß er durch die Öffnungen (108) in der Scheibe (106) paßt.

29. Blutprobenbehandlungsvorrichtung nach einem der Ansprüche 26 bis 28,
dadurch gekennzeichnet, daß die Messerhaltestation (46) einen beweglichen Messerhalter (70, 72, 74, 78, 80) zur Abstützung des Messers (10) hat, um das Messer (10) in einer vorbestimmten Position den Messertransportmitteln zuzuführen.

30. Blutprobenbehandlungsvorrichtung nach einem der Ansprüche 26 bis 29,
dadurch gekennzeichnet, daß die mindestens eine Reinigungs- und Desinfektionsstation folgendes umfaßt:
- eine erste Station (50, 52) mit einer Zuführeinrichtung für kaltes Wasser zum Reinigen des Messers (10) mit kaltem Wasser;
- eine zweite Station (54, 56) mit einer Zuführeinrichtung für Desinfektionsmittel zur Desinfektion des Messers (10);
- eine dritte Station (58) mit einer Spülvorrichtung zum Spülen des Messers (10) mit kaltem Wasser; und
- eine vierte Station (60) mit einer Luftblasvorrichtung zum Trokkenblasen des Messers (10) mit Luft.

31. Blutprobenbehandlungsvorrichtung nach Anspruch 30,
dadurch gekennzeichnet, daß die erste Station (50, 52) und die zweite Station (54, 56) jeweils zur inneren und äußeren Reinigung/Desinfektion des Messers (10) vorgesehen sind.

32. Blutprobenbehandlungsvorrichtung nach einem der Ansprüche 26 bis 31,
dadurch gekennzeichnet, daß die Messerzuführstation (66) einen beweglichen Messerhalter (70, 72, 74, 78, 80) aufweist, der das Messer (10) abstützt, wenn es von den Messertransportmitteln in eine vorbestimmte Position bewegt wird.

33. Blutprobenbehandlungsvorrichtung nach einem der Ansprüche 26 bis 30,
dadurch gekennzeichnet, daß die Messertransportmittel eine Greifvorrichtung (86) aufweisen, die das Messer (10) ergreift, um es während des Transports durch die Stationen in einer vorbestimmten Lage zu halten, wobei die Greifvorrichtung (86) zwischen einer offenen und einer geschlossenen Lage bewegbar ist.

34. Blutprobenbehandlungsvorrichtung nach einem der Ansprüche 26 bis 33,
dadurch gekennzeichnet, daß die Messertransportmittel eine Kreisbewegung ausführen, und daß die Stationen in vorbestimmten Winkelpositionen längs dieses Kreises angeordnet sind.

## Revendications

1. Procédé pour traiter le sang d'un animal individuel abattu, comprenant les étapes consistant à :
sectionner un faisceau sanguin de l'animal abattu de manière à évacuer le sang;
prélever un échantillon de sang (42) à partir du sang évacué;
analyser l'échantillon de sang,
caractérisé en ce qu'on prélève l'échantillon de sang (42) en pratiquant une incision dans l'animal (6) devant être abattu, dans un abattoir, à l'emplacement d'incision (12) sur l'animal devant être abattu, et qu'on collecte l'échantillon de sang (42) dans au moins un récipient à échantillon (36) qui est couplé à et est déplaçable avec un couteau (10) utilisé pour pratiquer l'incision, à la suite de quoi on détache le au moins un récipient à échantillon (36), du couteau (10), pour analyser l'échantillon de sang.

2. Procédé selon la revendication 1, caractérisé en ce que cet échantillon de sang (42) est collecté dans deux récipients à échantillons différents (36), qui sont couplés au couteau (10).

3. Procédé selon la revendication 1 ou 2, caractérisé en ce qu'après avoir détaché chaque récipient à échantillon (36) rempli de sang, du couteau (10), on nettoie et on désinfecte le couteau (10), puis on recouple un ou de nouveaux récipients à échantillons (36) au couteau.

4. Procédé selon la revendication 3, caractérisé en ce qu'on ferme de façon étanche un récipient à échantillon (36), qui a été rempli de sang lors de l'incision pratiquée dans l'animal (6) devant être abattu, après l'avoir détaché du couteau (10) et l'avoir amené dans une pièce (196), qui est située dans l'abattoir et dans laquelle une ou plusieurs analyses prédéterminées de l'échantillon de sang (42) sont effectuées.

5. Procédé selon la revendication 4, caractérisé en ce que le récipient à échantillon (36) est transféré dans un tuyau (124) ou un tube à l'aide d'un fluide qui est placé à une pression supérieure ou inférieure à la pression atmosphérique.

6. Procédé selon l'une quelconque des revendications 3-5, caractérisé en ce que le nettoyage et la désinfection du couteau (10) après que chaque récipient à échantillon (36) rempli de sang (42) a été détaché, comprend les étapes suivantes :
- nettoyage avec de l'eau froide,
- désinfection, notamment avec de l'eau à 82°C;
- rinçage à l'eau froide; et
- séchage par soufflage.

7. Procédé selon la revendication 6, caractérisé en ce qu'on nettoie le couteau (10) à l'eau froide et facultativement qu'on le fait sécher par soufflage avant de détacher chaque récipient à échantillon (36) rempli par le sang (42).

8. Procédé selon l'une quelconque des revendications 1-7, caractérisé en ce que chaque récipient à échantillon (36) rempli par du sang (42) est pourvu d'une marque d'identification du récipient à échantillon, qui est associée à une marque d'identification d'un animal devant être abattu, placé sur ou dans ledit animal (6) devant être abattu, d'où provient le sang situé dans le récipient à échantillon, la marque d'identification du récipient à échantillon et la marque d'identification de l'animal devant être abattu étant lues chacune moyennant l'utilisation d'un dispositif de lecture (131), et les données résultantes de cette lecture sont mémorisées en combinaison dans un fichier de données.

9. Procédé selon la revendication 8, caractérisé en ce qu'après l'analyse de l'échantillon de sang (42), des données associées aux résultats d'analyse sont ajoutées à ladite combinaison de données.

10. Procédé selon l'une quelconque des revendications 1-9, caractérisé en ce qu'un anticoagulant est introduit dans chaque récipient à échantillon (36) avant son utilisation.

11. Couteau pourvu d'un conduit (28) possédant une extrémité ouverte (27) dans la zone de la lame de couteau (22), qui est destinée à pénétrer dans un animal (6) devant être abattu, pour l'exécution d'une incision dans l'animal devant être abattu, lequel conduit (28) est relié à son autre extrémité d'une manière pouvant transmettre un liquide, à au moins un récipient à échantillon (36), qui est couplé à et est déplaçable avec le couteau, le au moins un récipient à échantillon (36) étant raccordé de façon détachable au couteau (10) et étant adapté pour contenir le sang d'un animal individuel devant être abattu.

12. Couteau selon la revendication 11, caractérisé en ce que le conduit (28) débouche, au niveau de ladite autre extrémité, dans une chambre intermédiaire (30), comportant au moins une sortie débouchant dans le au moins un autre récipient à échantillon (36).

13. Couteau selon la revendication 12, caractérisé en ce que la chambre intermédiaire (30) est pourvue d'au moins une ouverture (40) débouchant dans l'atmosphère et servant à ventiler et aérer la chambre intermédiaire.

14. Couteau selon la revendication 12 ou 13, caractérisé en ce que la chambre intermédiaire (30) comporte au moins une ouverture (40) débouchant dans l'atmosphère, pour la détermination un niveau maximum de sang dans la chambre intermédiaire.

15. Couteau selon l'une quelconque des revendications 12-14, caractérisé en ce que la sortie de la chambre intermédiaire comprend un embout tubulaire (32) et en ce que le récipient à échantillon (36) comprend un tube à échantillon possédant une extrémité fermée et une extrémité opposée ouverte, l'embout tubulaire et le tube à échantillon pouvant être repoussés l'un sur l'autre, et l'extrémité libre de l'embout tubulaire étant raccordée au tube à échantillon (34) établissant l'étanchéité.

16. Couteau selon l'une quelconque des revendications 11-15, caractérisé en ce que la lame de couteau (22) est pourvue d'un renfoncement (24) servant à loger au moins une partie d'un conduit agencé sous la forme d'un tube (28).

17. Couteau selon la revendication 16, caractérisé en ce que le conduit (28) est disposé de façon amovible dans le renfoncement (24).

18. Couteau selon la revendication 17, caractérisé en ce que le conduit (28) est fixé par adhérence à des parties saillantes (26) qui font saillie dans le renfoncement (24).

19. Couteau selon l'une quelconque des revendications 11-15, caractérisé en ce que la lame de couteau (204;205) est pourvue d'un trou (210) servant à loger une extrémité du conduit (28a).

20. Couteau selon l'une quelconque des revendications 11-15 ou 19, caractérisé en ce que la chambre intermédiaire (30) est fixée à la lame de couteau (204;205).

21. Couteau selon la revendication 20, caractérisé en ce que la fixation est réalisée au moyen d'un système de fixation à vissage.

22. Couteau selon la revendication 20, caractérisé en ce que la fixation est formée par un système de fixation à circlips (212,214).

23. Couteau selon la revendication 11-22, caractérisé par au moins une partie saillante (38) servant à positionner le couteau (10) dans un support de couteau (80;88), qui possède une forme au moins partiellement complémentaire de la au moins une partie saillante.

24. Couteau selon la revendication 23, caractérisé par deux parties saillantes (38) qui font saillie dans des directions opposées.

25. Couteau selon la revendication 23 ou 24, caractérisé en ce qu'une portion de chaque partie saillante (38) possède une section transversale essentiellement circulaire et que l'autre portion de la partie saillante possède une section transversale essentiellement rectangulaire.

26. Dispositif (14) de traitement d'un échantillon de sang servant à traiter un échantillon de sang (42) d'un animal abattu (6), comprenant :
- un poste (46) de retenue d'un couteau servant à retenir un couteau (10) selon l'une quelconque des revendications 11-25;
- un poste (62) de retrait du ou des récipients à échantillons servant à retirer le ou les récipients à échantillons (36) à partir du couteau (10);
- au moins un poste de nettoyage et/ou de désinfection (48,50,52,54,56,58,60) pour nettoyer et désinfecter le couteau (10);
- un poste (64) de fixation du ou des récipients à échantillons servant à fixer le ou les récipients à échantillons (36) au couteau (10);
- un poste (66) de délivrance du couteau servant à délivrer le couteau (10); et
- des moyens de transport du couteau servant à transporter le couteau (10) devant les postes.

27. Dispositif de traitement d'un échantillon de sang selon la revendication 26, caractérisé par des moyens de transport du ou des récipients à échantillons comprenant un dispositif de serrage (116), qui est disposé sur un bras mobile (112,114) servant à saisir au moins un récipient à échantillon (36), ainsi qu'un disque rotatif (106), qui comporte des ouvertures, servant à retenir le ou les récipients à échantillons (36) et à fixer / retirer de petits capuchons (110) sur / à partir dudit récipient, et au moins un tuyau (124,126) ou un tube, dans lequel le ou les récipients à échantillons (36) peuvent être transportés à une pression inférieure à la pression atmosphérique ou à une pression supérieure à la pression atmosphérique, le bras (112,114) équipé du dispositif de serrage (116) transportant le ou les récipients à échantillons (36) à partir du poste (62) de retrait du ou des récipients à échantillons en direction du disque (106), et le disque (106) convoyant le ou les récipients à échantillons (36) depuis le bras (112,114) équipé du dispositif de serrage (116) jusqu'au un ou plusieurs tuyaux (124) ou tubes ou sinon le transport s'effectuant en sens opposé, depuis le ou les tuyaux (126) ou tubes en direction du poste (64) de fixation du ou des récipients à échantillons.

28. Dispositif de traitement d'un échantillon de sang selon la revendication 27, caractérisé en ce que le petit capuchon (110) possède des dimensions qui sont telles qu'il ne peut pas passer dans les ouvertures (108) formées dans le disque (106), alors que le récipient à échantillon (36) possède des dimensions qui sont telles qu'il peut passer dans les ouvertures (108) aménagées dans le disque (106).

29. Dispositif de traitement d'un échantillon de sang selon l'une quelconque des revendications 26-28, caractérisé en ce que le poste (46) de retenue du couteau comporte un support mobile de couteau (70,72,74,78,80) servant à supporter le couteau (10) pour amener le couteau (10) jusqu'aux moyens de transport du couteau, dans une position prédéterminée.

30. Dispositif de traitement d'un échantillon de sang selon l'une quelconque des revendications 26-29, caractérisé en ce que le au moins un poste de nettoyage et de désinfection comprend :
- un premier poste (50,52) comportant un dispositif d'alimentation en eau froide pour nettoyer le couteau (910) avec de l'eau froide;
- un second poste (54,56) comportant un dispositif d'alimentation en désinfectant servant à désinfecter le couteau (10);
- un troisième poste (58) comportant un dispositif de rinçage servant à rincer le couteau (10) avec de l'eau froide; et
- un quatrième poste (60) comportant un dispositif de soufflage d'air servant à sécher par soufflage le couteau (10) en utilisant de l'air.

31. Dispositif de traitement d'un échantillon de sang selon la revendication 30, caractérisé en ce que le premier poste (50,52) et le second poste (54,56) sont conçus chacun pour le nettoyage / la désinfection interne et externe du couteau (10).

32. Dispositif de traitement d'un échantillon de sang selon l'une quelconque des revendications 26 à 31, caractérisé en ce que le poste (66) de délivrance du couteau comprend un support mobile de couteau (70,72,74,78,80) servant à supporter le couteau (10) pour retirer le couteau (10) depuis les moyens de transport du couteau, pour l'amener dans une position prédéterminée.

33. Dispositif de traitement d'un échantillon de sang selon l'une quelconque des revendications 26-32, caractérisé en ce que les moyens de transport du couteau comprennent un dispositif de préhension (86), qui s'engage sur le couteau (10) pour fixer le couteau (10) dans une position prédéterminée pendant le transport du couteau (10) devant les postes, lequel dispositif de préhension (86) peut être déplacé entre une position fermée et une position ouverte.

34. Dispositif de traitement d'un échantillon de sang selon l'une quelconque des revendications 26-33, caractérisé en ce que les moyens de transport du couteau exécutent un déplacement circulaire et en ce que les postes sont disposés dans des positions angulaires prédéterminées le long du cercle.
